# EUROPEAN PATENT APPLICATION

(11) **EP 2 258 406 A2**
(43) Date of publication of application: **08.12.2010**
(21) Application number: 10158698.0
(22) Date of filing: 31.03.2010
(51) Int. Cl.: A61L 15/22

(54) **Scar treatment silicone dressing**

(30) Priority: 21.04.2009 TW 098113147
(71) Applicant: Fortune Medical Instrument Corp., Danshuei Jen T'ai pei (TW)
(72) Inventor: Lu, Yu-Ping, Taipei Hsien (TW); Wang, Tyler, Jhong-Li City (TW)
(74) Representative: Dossmann, Gérard

(57) **Abstract**

A scar treatment silicone dressing used for attaching onto wound of skin, comprising: a surface layer, a connection layer, a silicone layer, and a protection film. The surface layer is moisture permeable and water resistant thus providing protection function, and on one of its sides is disposed a silicone layer through a connection layer. The silicone layer is provided with penetrating through holes for controlling dissipation of moisture of skin wounds, as such assisting skin exiting perspiration to said surface layer via said through holes, hereby reducing the possibility that the acidic metabolite in perspiration getting in touch with the skin for long period of time in causing irritations / inflammations of the skin; meanwhile, it is also capable of preventing over-dissipation of moisture from the surface of skin, thus it will not adversely affect the repair and reconstruction of the scar tissue.

## Description

The present invention relates to a dressing, and in particular to a scar treatment silicone dressing.

In general, in a healing process of an open wound of a skin, especially in a period of from the fourth week to the sixth month after the infliction of the wound, in case that the wounds are not treated properly, then the collagen fibers that are not able to maintain the same alignment with the normal skin are very likely to form scar, and accompany hypertrophic scar or keloid, such that the abnormal growth of this kind of skin tissue tends to create changes to the color and shape of skin around the close wounds area after healing-up of the wound, thus adversely affecting the appearance of the inflictor.

In this respect, for many years, silicone dressing has been utilized to treat the reddish and protrusion scar by the medical profession. As such, silicone dressing is used to press against the newly grown skin at the healed-up wounds, such that the pressure produced is used to deter the irregular growth of the scar, moreover, its hydrophobic property is able to prevent the evaporation of moisture at the skin surface of the scar, therefore, for the newly healed-up skin, it is capable of reducing the deposition of collagen cell, and controlling the additional growth of scar; while for the old scar, the moisture contained in the stratum corneum of the skin may increase, such that the water soluble compounds in the sear are able to diffuse to its surface, hereby reducing the fluid pressure, therefore, the sear tends to be softened and be flexible, then it is naturally liable to be repaired and to improve its appearance. In addition, due to the low adhesion of the silicone layer, thus in taking it off from the skin, the mechanical damage to the skin as caused by undue peeling forces can be avoided. Furthermore, due to the soft texture, light weight, thin structure, and washable characteristics of the silicone dressing, it can be reused and self-adhesive to various scar wounds of the body easily, thus providing enormous medicare convenience to the home nursing care.

However, since during the healing process, a silicone dressing has to be attached and adhered to the scar for long period of time (usually, about 1 to 6 months), such that the silicone dressing will deter the dissipation of perspiration and temperature, as such, due to the long period close contact with the silicone dressing, the skin is liable to develop various irritations / inflammations, such as itches and rashes. Therefore, the effectiveness and efficacy of silicone dressing of the prior art are not quite satisfactory, and it has much room for improvement.

In view of the problems and shortcomings of the prior art, a major objective of the present invention is to provide a scar treatment silicone dressing, wherein, special emboss design is utilized to control the dissipation of moisture at skin wounds, such that perspiration of the skin is exited to the surface layer via the through holes, thus reducing the time the acidic metabolite of perspiration getting in touch with the skin in causing irritations / inflammations of the skin. Besides, this kind of scar treatment silicone dressing is able to prevent the over dissipation of moisture from the surface of the skin, thus it will not adversely affect the healing up and repair of the scar.

In order to achieve the above mentioned objective, the present invention provides a scar treatment silicone dressing, comprising: a silicone layer, a surface layer, a connection layer, and a protection film. The silicone layer is provided with at least a penetrating through hole, the surface layer is connected to the silicone layer through the connection layer, and is in communication with the through hole of the silicone layer, so as to protect the silicone layer. Therefore, when a user attaches the scar treatment silicone dressing onto his skin, and in the long period of application, the perspiration and temperature normally produced by the skin can be dissipated to the surface layer via the through hole, so as to remove the acidic metabolite of perspiration away from the surface of the skin, thus preventing the irritations / inflammations as caused by long period contact of the skin. Moreover, the special design of the through hole is able to prevent the over dissipation of moisture from the surface of the skin, thus it will not adversely affect the healing up and repair of the scar.

As mentioned above, the scar treatment silicone dressing of the present invention is further provided with a protection film, that is attached to the reverse side of the silicone layer, such that, under normal conditions, it can not only protect the silicone layer, but when it is connected to the silicone layer, it can also press directly against the silicone layer to form the through holes required by means of the special embosses of the protection film, hereby achieving the effect of creating and maintaining the through holes in the silicone layer.

Further scope of the applicability of the present invention will become apparent from the detailed descriptions given hereinafter. However, it should be understood that the detailed descriptions and specific examples, while indicating preferred embodiments of the present invention, are given by way of illustration only, since various changes and modifications within the spirit and scope of the present invention will become apparent to those skilled in the art from this detailed descriptions.

The related drawings in connection with the detailed descriptions of the present invention to be made later are described briefly as follows, in which:

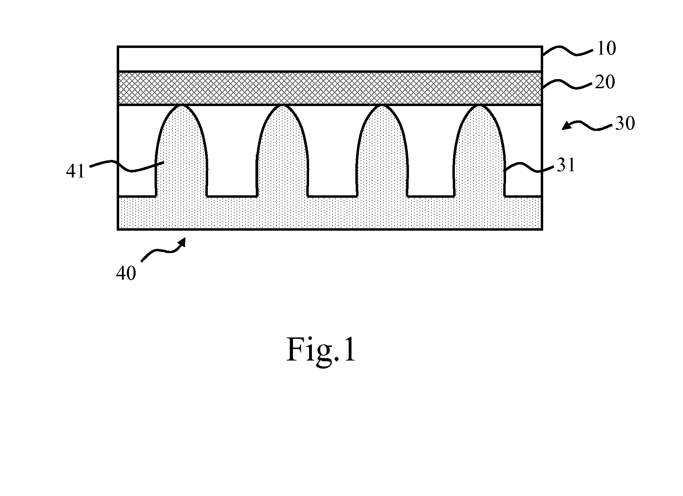

Fig.1 is a cross section view of a scar treatment silicone dressing according to an embodiment of the present invention.

The purpose, construction, features, functions and advantages of the present invention can be appreciated and understood more thoroughly through the following detailed description with reference to the attached drawings.

The present invention provides a scar treatment silicone dressing. Firstly, refer to Fig. 1 for a cross section view of a scar treatment silicone dressing according to an embodiment of the present invention.

As shown in Fig. 1, the scar treatment silicone dressing of the present invention is composed of a surface layer 10, a connection layer 20, a silicone layer 30, and a protection film 40. In the following, the structure and function of each of the layers mentioned above will be described in detail.

In the structure of scar treatment silicone dressing mentioned above, the outer most layer is a surface layer 10, that is made of a moisture permeable and water resistant material, such as plastic, rubber, multi-layer composite films, natural or artificial leather, high density woven or non-woven, etc. The surface layer 10 is well friction resistant, thus it is placed on the silicone layer 30 which is poor friction resistant and is adhesive, so as to protect the silicone layer 30 from the damage of friction as caused by clothing or external force. In addition, the surface layer 10 has the advantages of being moisture permeable, water resistant, soft texture, and easily flexible, so as to make the silicone dressing more convenient and comfortable in application. In the present invention, PU film is utilized for the surface layer 10, with its thickness of 0.01--0.10mm, preferably 0.02±0.01mm.

The connection layer 20 is disposed and connected between the surface layer 10 and the silicone layer 30. Since the adhesion between the surface layer 10 and the silicone layer 30 is not quite satisfactory, therefore, in order to avoid adhesion failure that could result in separation of the surface layer 10 and the silicone layer 30, the following materials can be selected in assisting adhesion: appropriate primer, such as organic coupling agent; or porous materials having large specific surface area, such as a porous nonwoven film. In the present embodiment, porous material having large specific surface area such as the nonwoven film is utilized, so as to avoid that the application of organic coupling agent might cause damage to the skin. Such a kind of nonwoven film is provided with a plurality of air ventilating holes and having better adhesion with the surface layer 10 and the silicone layer 30, thus it is able to improve the adhesion between the two layers, and it is in communication with the through holes 31 of the silicone layer 30 (this will be described in detail later), thus facilitating exiting of the perspiration from the skin, hereby preventing the irritations / inflammations of the skin as caused by its long period contact with the acidic metabolite in the perspiration. In addition, its soft and easily flexible characteristics will render comfort in application of the scar treatment silicone dressing.

The silicone layer 30 is provided with a plurality of through holes 31 thus exposing parts of the connection layer 20, such that these through holes 31 can be disposed in arrangement of equal intervals or in irregular arrangement, and having its shape such as round shape, polygons, numbers, characters, hills and valleys, or other creative shapes. The density of the through holes in the silicone layer 30 is about 1-100 through holes/ cm², preferably in the range of 10--30 through holes/ cm². In addition to being used to press against the newly grown skin at the healed-up wounds, the silicone layer 30 is able to prevent the growing outward of the scar, also, its hydrophobic property is able to deter the dissipation of moisture from the skin surface of the scar, and the through hole 31 may control the moisture dissipation speed in a range of transepidermal water loss (TEWL) (the differences between persons of different races are minimal, averaging about 20g/m²/hr).

In the present embodiment, the silicone layer 30 is designed to have low adhesion, with its peeling strength in a range of 0.2∼1.0N/inch, preferably in a range of 0.7±0.1N/inch (according to a test method of American Society for Testing and Materials ASTM D3330). Moreover, the silicone layer 30 is designed to have high holding power, and that is greater than 50 min/inch²/200g at room temperature (according to a test method of American Society for Testing and Materials ASTM D3654; in order to match the low adhesion of silicone dressing, the test weight is changed from 1Kg to 200g). In addition, the silicone layer 30 is designed to have limited water vapor permeation, with its water vapor permeation being controlled in a range of transepidermal water loss (TEWL): 20±10g/m²/h (according to test method of American Society for Testing and Materials ASTM E96).

In the structure mentioned above, the protection film 40 can be made of plastic, rubber, paper, or specially processed release film, etc, such that it is able to protect the silicone layer 30 from being contaminated under normal condition, and it can be peeled off upon usage. The protection film 40 is provided with special embosses, that are in turn provided with a plurality of protrusion portions 41 corresponding to the through holes 31, such that when they are combined with the silicone layer 30, the protrusion portions 41 can extrude the silicone layer 30 directly to form the through holes 31, so as to facilitate exiting the perspiration to the surface layer 20 in preventing the irritations / inflammations of the skin. In the present invention, the protection film 40 is made of PE.

In the present embodiment, the overall width for the scar treatment silicone dressing disclosed is 0.2∼2.0mm, preferably 0.3±0.05mm, and with its water resistance > 10000mmH₂O(According to the Test Method of American Association of Textile Chemists and Colorists AATCC 127), and with its water repellency > 80 % (According to the Test Method of American Association of Textile Chemists and Colorists AATCC 22 ).

Therefore, in applying the scar treatment silicone dressing onto the healing wound of the inflictor's skin, it can easily deter the growth of the scar, such that in a long period of application, the perspiration produced by the skin is able to infiltrate to the surface layer via the through hole of the silicone layer, so as to avoid long period of contact of acidic metabolite of perspiration with the skin in causing irritations / inflammations of the skin. As such, the special design of through hole is able to control the dissipation of water vapor from the surface of the skin, thus it can modify, reduce, soften, de-color, and then gradually eliminate the old and new scars in improving the overall appearance of the wound portion of the inflictor's skin.

The above detailed description of the preferred embodiment is intended to describe more clearly the characteristics and spirit of the present invention. However, the preferred embodiments disclosed above are not intended to be any restrictions to the scope of the present invention. Conversely, its purpose is to include the various changes and equivalent arrangements which are within the scope of the appended claims.

## Claims

1. A scar treatment silicone dressing, comprising
a silicone layer, provided with at least a penetrating through hole;
a connection layer, disposed and connected on said silicone layer, and is made of
organic coupling agent or porous materials having large specific surface area; and
a surface layer, disposed and connected on said connection layer, so as to protect
said connection layer and said silicone layer.

2. The scar treatment silicone dressing as claimed in claim 1, further comprising:
a protection film, disposed and attached on a reverse side of said silicone layer, and is
used to protect said silicone layer; wherein
said protection film is made of embossed PE film.

3. The scar treatment silicone dressing as claimed in claim 2, wherein
said protection film is provided with a protrusion portion corresponding to said through hole of said silicone layer.

4. The scar treatment silicone dressing as claimed in claim 1, wherein
said silicone layer is provided with said plurality of through holes, disposed in equal
interval or irregular arrangement; wherein
said plurality of through holes are of round shape, polygons, numbers, characters, or hills and valleys shape.

5. The scar treatment silicone dressing as claimed in claim 4, wherein
a density of said plurality of through holes of said silicone layer is about 1--100 through holes / cm².

6. The scar treatment silicone dressing as claimed in claim 5, wherein
said density of said plurality of through holes of said silicone layer is about 10--30 through holes / cm².

7. The scar treatment silicone dressing as claimed in claim 1, wherein
said connection layer is a porous nonwoven film.

8. The scar treatment silicone dressing as claimed in claim 1, wherein
said surface layer is made of a moisture permeable and water resistance material;
wherein
said moisture permeable and water resistance material is selected from a group comprising of plastic, rubber, multi-layer composite films, natural leather, artificial leather, high density woven and non-woven.

9. The scar treatment silicone dressing as claimed in claim 8, wherein
said surface layer is made of a PU film.

10. The scar treatment silicone dressing as claimed in claim 8, wherein
a thickness of said surface layer is 0.01∼0.10mm.

11. The scar treatment silicone dressing as claimed in claim 10, wherein
said thickness of said surface layer is 0.02±0.01mm.

12. The scar treatment silicone dressing as claimed in claim 1, wherein
a total thickness of said scar treatment silicone dressing is 0.2∼2.0mm.

13. The scar treatment silicone dressing as claimed in claim 12, wherein
said total thickness of said scar treatment silicone dressing is 0.3 ± 0.05mm.

14. The scar treatment silicone dressing as claimed in claim 1, wherein
adhesion of said silicone layer is 0.2∼0.1N/inch as determined by the American Society for Testing and Materials ASTM D3330; wherein
holding power of said silicone layer is greater than 50 min/inch²/200g at room temperature as determined by the American Society for Testing and Materials ASTM D3654; in order to match low adhesion of said silicone dressing, test weight is changed from 1Kg to 200g; wherein
water vapor permeation of said silicone layer is controlled in a range of transepidermal water loss (TEWL): 20±10g/m²/hr as determined by the American Society for Testing and Materials ASTM E96.

15. The scar treatment silicone dressing as claimed in claim 14, wherein
adhesion of said silicone layer is 0.7±0.1N/inch as determined by the American Society for Testing and Materials ASTM D3330.
